# EUROPÄISCHE PATENTANMELDUNG

(11) **EP 3 569 204 A1**
(43) Veröffentlichungstag der Anmeldung: **20.11.2019**
(21) Anmeldenummer: 19162193.7
(22) Anmeldetag: 12.03.2019
(51) Int. Cl.: A61F 5/56, A61F 5/08, A61F 2/18, A61B 17/24, A62B 23/06, A61M 15/08

(54) **PHONATIONSVENTIL**

(30) Priorität: 18.05.2018 DE 102018112093
(71) Anmelder: IFA3D Medical Solutions GmbH, 12587 Berlin (DE)
(72) Erfinder: VELTEN, Andreas, 12587 BERLIN (DE)
(74) Vertreter: Müller, Wolfram Hubertus

(57) **Zusammenfassung**

Die Erfindung betrifft ein Phonationsventil für die nasalen Atemwege umfassend ein ringförmiges Tragelement (1), das auf seiner unteren Seite (5) flanschartig auslaufend ausgebildet ist und welches ein Stützgerüst (2) zur Aufnahme und Stabilisierung einer Membran (3) aufweist, die einen temporären Verschluss des Phonationsventils beim Ausatmen bildet und ein ungehindertes Einatmen ermöglicht.

## Beschreibung

Die Erfindung betrifft ein Phonationsventil, das bei funktionellen Störungen des Gaumensegels beim Menschen zur Anwendung kommen kann.

Das aus Muskeln, Bindegewebe und Schleimhaut bestehende Gaumensegel grenzt zusammen mit dem Zungengrund die Mundhöhle gegen den Rachen ab. Die wesentlichen Funktionen des Gaumensegels bestehen in der Lautbildung sowie in der Abtrennung von Speiseröhre und Atemwegen während des Schluckens und des Sprechens. Auf diese Weise soll verhindert werden, dass Speisebrei, Essensbestandteile oder Flüssigkeiten unbeabsichtigt in die Nasenhöhle und die Atemwege gelangen können und die Lautbildung bei oralen Lauten und Nasalvokalen nicht behindert wird.

Eine schlaffe Gaumensegelmuskulatur oder andere Ursachen, insbesondere Fehlbildungen des Gaumensegels, können aufgrund der artikulatorischen Funktion des Gaumensegels bei der betroffenen Person die Phonation behindern und beispielsweise Schwierigkeiten oder Ungenauigkeiten beim Sprechen hervorrufen. Auch das Schnarchen, Schlafatemgeräusche und Schlafapnoe werden mit zu den häufigsten Beschwerden in Verbindung mit dem Gaumensegel gerechnet.

Bislang wird den Betroffenen mit Störungen der artikulatorischen Funktion des Gaumensegels empfohlen, neben einer logopädischen Sprachtherapie bei anhaltender Symptomatik die Nase manuell zu verschließen um so die Lautbildung zu ermöglichen.

Alternativ kann mittels einer Nasenklammer, wie zum Beispiel aus DE 299 138 92 U1 oder WO 2009/056562 WO hervorgeht, die Nase verschlossen werden, um den für die Lautbildung erforderlichen Druckaufbau zu ermöglichen. Des Weiteren werden operative Techniken eingesetzt um Fehlfunktionen des Gaumensegels zu beheben.

Hier setzt die vorliegende Erfindung an, deren Aufgabe es ist, einen auf die individuellen anatomischen und physischen Bedingungen des Betroffenen angepassten und einfach handhabbaren temporären Verschluss für die nasalen Atemwege bereitzustellen, der das Ausatmen (Expiration) ermöglicht und der die Einatmung (Inspiration) nicht behindert.

Erfindungsgemäß wird die Aufgabe durch ein Phonationsventil für die nasalen Atemwege gelöst, das ein ringartig ausgebildetes Tragelement, das auf einer Seite flanschartig auslaufend ausgebildet ist und welches ein Stützgerüst zur Aufnahme und Stabilisierung einer Membran aufweist, die einen temporären Verschluss des Phonationsventils beim Ausatmen sichert und ein ungehindertes Einatmen ermöglicht.

Mit der Erfindung verbindet sich der besondere Vorzug, dass erstmals ein Ventil bereitgestellt wird, das den individuellen anatomischen und physischen Bedingungen des Betroffenen angepasst ist. Es lässt sich einfach handhaben und ohne Probleme von dem Betroffenen in das Nasenloch einsetzen und auch wieder daraus entfernen. Durch die Ausformung des Ventils exakt nach der inneren Kontur des Nasenloches gibt die Erfindung dem Betroffenen auch einen hohen Tragekomfort.
Des Weiteren wird durch den exakten Sitz eine umfängliche Abdichtung bei der Expiration erreicht, welche die Bildung des für die Lautbildung gewünschten Überdruck im Nasen-Rachen-Raum gewährleistet.

Besonders bevorzugt ist das Tragelement des Phonationsventils der inneren Kontur eines Nasenvorhofes eines Nasenloches angepasst.

Typischerweise ist das Stützgerüst gitterförmig ausgebildet und ist die Membran mit dem gitterförmigen Stützgerüst bevorzugt zentral verbunden.

Vorzugsweise kann das Stützgerüst mindestens einen Steg oder mehrere parallel zueinander angeordnete Stege zur Aufnahme der Membran besitzen.

Gemäß einer bevorzugten Ausführungsform der Erfindung ist die Membran mittels Verrasten oder Verkleben mit dem gitterförmigen Stützgerüst oder einem Steg oder mehreren Stegen verbunden.

Vorteilhafterweise ist das Stützgerüst mittels eines medizinisch zugelassenen Haftvermittlers mit dem Tragelement abdichtend verbunden.

Eine weitere Ausführungsform der Erfindung sieht vor, dass die Membran aus einem Medical Grade Silikon und/oder vergleichbaren Softmaterialien in der Härte von 20 Shore A bis 50 Shore A besteht und eine Dicke von 0.5 mm bis 1.0 mm aufweist.

Typischerweise ist zwischen dem Rand der Membran und dem inneren Rand des Tragelementes ein Spalt vorgesehen.

Vorzugsweise besteht das Stützgerüst aus einem medizinisch zugelassenem Polymer oder einem Medical Grade Silikon mit einer Shore-Härte von 50 Shore A bis 20 Shore D.

Besonders bevorzugt besteht das Tragelement aus einem Silikon medizinischer Qualität mit einer Shore-Härte von 50 Shore A bis 20 Shore D.

Eine besonders bevorzugte Ausführungsform sieht vor, das Phonationsventil im 3D-Druck herzustellen.

Im Folgenden soll die Erfindung an Hand von Zeichnungen näher erläutert werden. Es zeigen:
- **Figur 1**: die Sicht auf ein Phonationsventil von unten in perspektivischer Darstellung;
- **Figur 2**: die Draufsicht auf das erfindungsgemäße Ventil ebenfalls in perspektivischer Darstellung;
- **Figur 3**: die Draufsicht auf ein Stützgerüst des Phonationsventils in perspektivischer Darstellung;
- **Figur 4**: das Stützgerüst gemäß **Fig. 3** im Schnitt A - A;
- **Figur 5**: den Längsschnitt eines Phonationsventils in perspektivischer Darstellung;
- **Figur 6**: die Sicht auf ein in ein Nasenloch eingesetztes Phonationsventil in perspektivischer Darstellung.

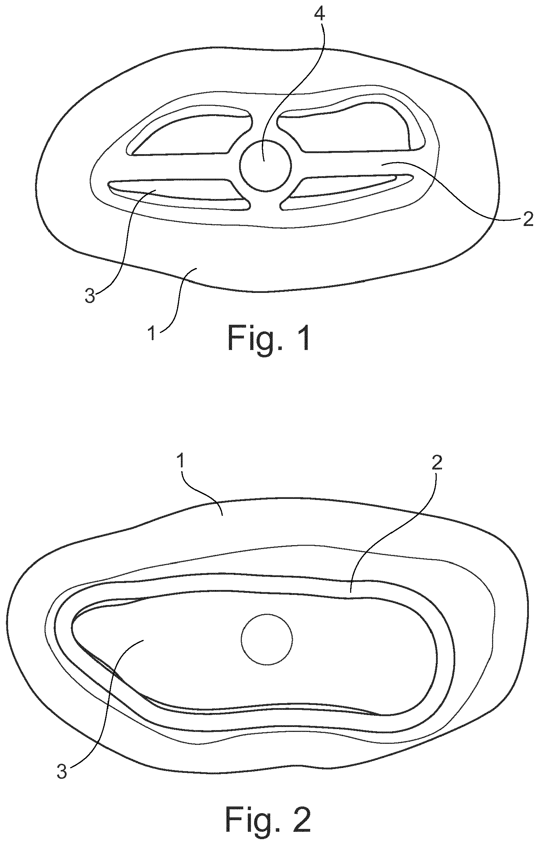

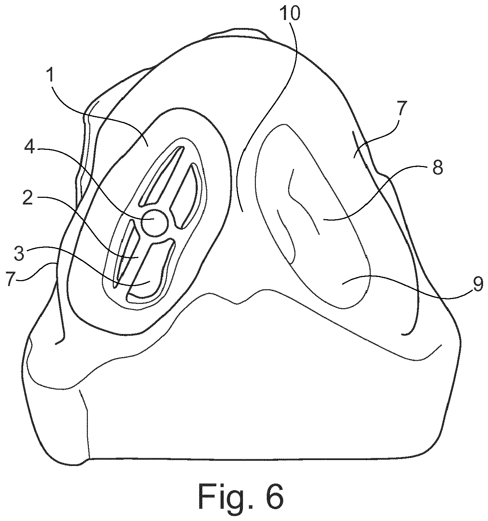

Aus **Fig. 1** und **Fig. 2** sind als Hauptbestandteile des Phonationsventils das ringartig ausgebildete Tragelement 1, das gitterförmige Stützgerüst 2 und die Membran 3 ersichtlich. Das Stützgerüst 2 ist vorzugsweise durch einen medizinisch zugelassenen Haftvermittler hier fest und abdichtend mit dem Tragelement 1 verbunden.
Als medizinisch zugelassener Haftvermittler kann ein Primer, zum Beispiel Softreliner Tough der Firma Tokuyama, eingesetzt werden.
Die Ausformung des Tragelementes 2 entspricht exakt der inneren Kontur des Nasenvorhofes 8 (siehe **Fig. 6**) des Nasenloches 9 und kann in seiner Formgebung entweder durch Abformung des Nasenraumes oder durch eine digitale Bildgebung (Endoskopie, CT, MRT, DVT) erzeugt werden.
Die Modulation bzw. Konstruktion des Ventils erfolgt dann mittels CAD-Programm.
Die Membran 3 ist bei diesem Ausführungsbeispiel durch die Verrastung 4 lösbar mit dem Stützgerüst 2 verbunden.
Das Tragelement 1 besteht, um einen hohen Tragekomfort zu gewährleisten, aus einem weichen Material, vorzugsweise einem Silikon medizinischer Qualität. Im vorliegenden Beispiel wurde dafür Odontosil der Firma Dreve Dentamid eingesetzt. Im vorliegenden Beispiel besitzt das Material eine Shore-Härte von 50 Shore A. Sie kann vorteilhafterweise im Bereich von 50 Shore A bis 20 Shore D liegen.
Das Tragelement 1 ist an seinem Rand flanschartig auslaufend ausgebildet um das Einsetzen des Phonationsventils in das Nasenloch 9 und das Entfernen des Ventils zu erleichtern.

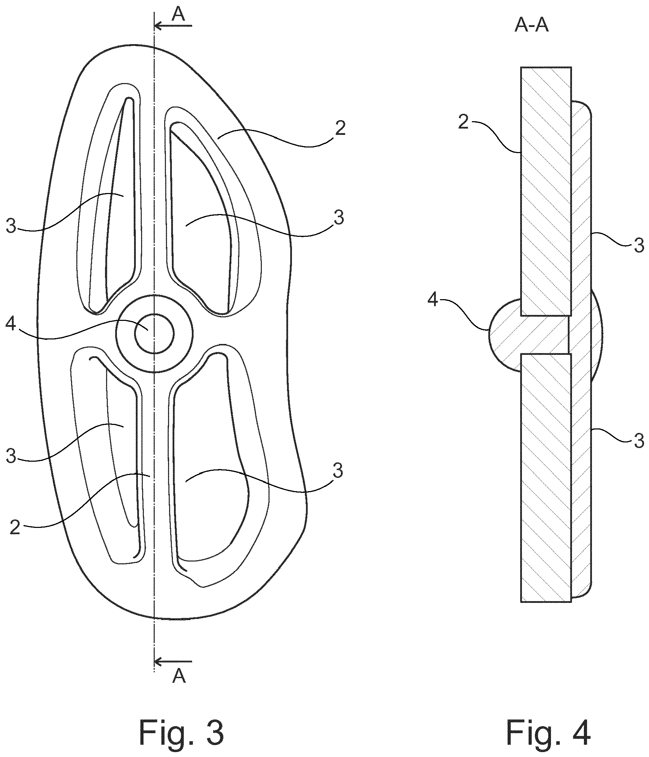

**Fig. 3** zeigt die Sicht auf ein gitterförmiges Stützgerüst 2 mit der Verrastung 4 für die Membran 3. Das Stützgerüst 2 besteht gewöhnlich aus einer härteren medizinisch zugelassenen Komponente, einem Photopolymer oder einem anderen harten Kunststoff, die für eine erhöhte Stabilität sorgen. Insbesondere bei einer größeren Dimensionierung des Ventils ermöglicht diese Komponente eine sichere Abstützung der Membran 3 auf dem Stützgerüst 2 wie dies zum Druckaufbau erforderlich ist. Als härtere Komponente, deren Shore-Härte im Bereich von 50 Shore A bis 20 Shore D liegen sollte, wurde bei diesem Ausführungsbeispiel Odontosil / Med 610 der Firmen Dreve Dentamid / Stratasys eingesetzt. Wenn es die Platzverhältnisse zulassen kann das Phonationsventil insgesamt mit all seinen Komponenten auch aus einem weichen elastischen Material gefertigt werden.
Statt einer gitterförmigen Struktur kann das Stützgerüst 2 auch einen oder mehrere parallel zueinander angeordnete hier nicht dargestellte Stege aufweisen, mit denen die Membran 3 fest oder lösbar verbunden ist.

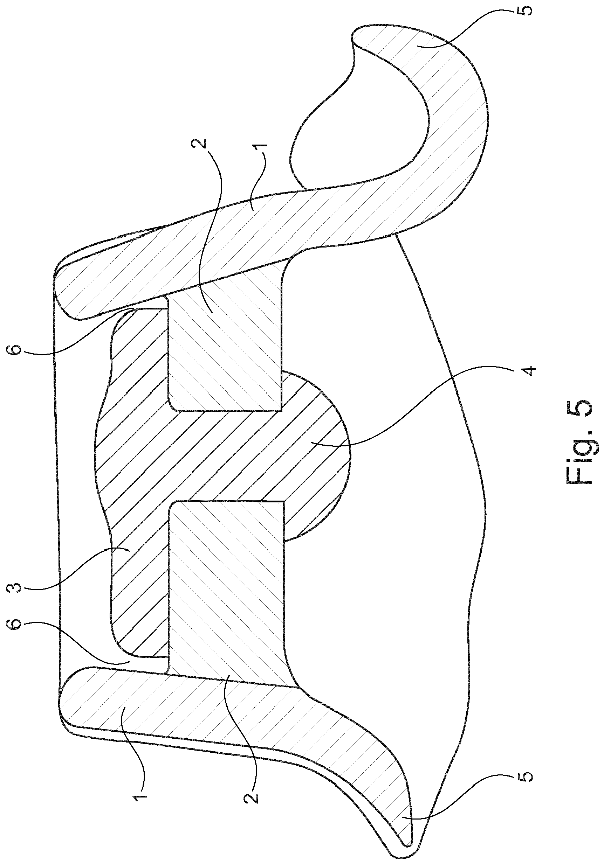

In **Fig. 4** ist im Schnitt A - A das Stützgerüst 2 der **Fig. 3** dargestellt. Deutlich ist zu erkennen, dass die Membran 3 nicht die gesamte Fläche des Stützgerüstes 2 bedeckt. Dadurch soll gewährleistet werden, dass die Membran 3 im eingebauten Zustand des Stützgerüstes 2 ungehindert das Einatmen und das Schließen des Ventils ermöglichen kann. Ohne den Spalt 6, wie er aus **Fig. 5** hervorgeht, besteht die Gefahr, dass die Bewegung der Membran 3 durch das angrenzende Tragegestell 1 behindert, wenn nicht sogar unmöglich wird.
Die Membran 3 besteht hier aus Lab-Air Silikon 25 der Firma Dreve Dentamid. Sie kann aus medizinisch zugelassenen Polymeren unterschiedlicher Härten gefertigt sein, um die beim Einatmen zu überwindende Druckschwelle einstellen zu können. Bewährt haben sich Materialien mit einer Shore-Härte von 20 Shore A bis 50 Shore A, abhängig von der Wandstärke.

**Fig. 5** zeigt schematisch in perspektivischer Darstellung das erfinderische Phonationsventil im Längsschnitt.
Die Membran 3 ist auch bei diesem Ausführungsbeispiel durch die Verrastung 4 mit dem Stützgerüst 2 verbunden und kann dadurch erforderlichenfalls auch gewechselt und gegebenenfalls durch eine Membran 3 mit einer anderen Shore-Härte getauscht werden. Deutlich ist der Spalt 6 zwischen der Membran 3 und dem Tragelement 1 zu erkennen, der eine ungehinderte Bewegung der Membran zum Öffnen und Schließen des Phonationsventils in Verbindung mit dem Überstand des Tragelementes 1 gegenüber dem Stützgerüst 2 zulässt. Die flanschartige Ausbildung 5 des Randes des Tragelementes 1 erweist sich insbesondere für das Einsetzen und Entfernen des Ventils als vorteilhaft.

Aus **Fig. 6** geht ein in den Nasenvorhof 8 eingesetztes Phonationsventil hervor. Das Ventil liegt so an der vorderen Schleimhaut der Nasenscheidewand 10 an, endet an der Außenseite der Nasenolive. Es wird durch das Nasenloch 9 in den Nasenvorhof 8 eingebracht, fixiert sich durch die im Nasenbereich vorhandenen Unterschnitte selbst und wird durch den Flügelknorpel 7 gehalten.
Um Verwechslungen der Phonationsventile hinsichtlich des für sie zutreffenden Nasenloches 9 zu vermeiden, können die Phonationsventile entsprechend gekennzeichnet werden

Das erfinderische Phonationsventil kann aus einem transparenten oder einem der Hautfarbe des Betroffenen angepassten Material im 3D-Druck gefertigt werden.

Als Druckverfahren können sowohl das Polyjet-Verfahren, als auch das Digital Light Projection (DLP), Stereolithografie (SLA), Laser Sinter Melting (SLM), sowie alle anderen hochauflösenden (bis max. 20 my) Druck- Verfahren Anwendung finden. Grundsätzlich dürfen hierbei jedoch nur medizinisch zugelassene Materialien verwendet werden.

### Bezugszeichenaufstellung

- 1: Tragelement
- 2: Stützgerüst
- 3: Membran
- 4: Verrastung
- 5: Flanschartige Ausbildung
- 6: Spalt
- 7: Knorpel
- 8: Nasenvorhof
- 9: Nasenloch
- 10: Nasenscheidewand

## Patentansprüche

1. Phonationsventil für die nasalen Atemwege umfassend ein ringförmiges Tragelement (1), das auf seiner unteren Seite (5) flanschartig auslaufend ausgebildet ist und welches ein Stützgerüst (2) zur Aufnahme und Stabilisierung einer Membran (3) aufweist, die einen temporären Verschluss des Phonationsventils beim Ausatmen bildet und ein ungehindertes Einatmen ermöglicht.

2. Phonationsventil nach Anspruch 1, **dadurch gekennzeichnet, dass** das Tragelement (1) der inneren Kontur des Nasenvorhofes 8 des Nasenloches (9) angepasst ist.

3. Phonationsventil nach Anspruch 1 und 2, **dadurch gekennzeichnet, dass** das Stützgerüst (2) gitterförmig ausgebildet und die Membran (3) mit dem gitterförmigen Stützgerüst (2) verbunden ist oder das Stützgerüst (2) weist mindestens einen Steg zur Aufnahme der Membran (3) auf.

4. Phonationsventil nach mindestens einem der Ansprüche 1 bis 3, **dadurch gekennzeichnet, dass** die Membran (3) mittels Verrasten (4) oder Verkleben mit dem gitterförmigen Stützgerüst (2) oder einem Steg oder mehreren Stegen verbunden ist.

5. Phonationsventil nach mindestens einem der Ansprüche 1 bis 4, **dadurch gekennzeichnet, dass** das Stützgerüst (2) mittels eines medizinisch zugelassenen Haftvermittlers mit dem Tragelement (1) abdichtend verbunden ist.

6. Phonationsventil nach mindestens einem der Ansprüche 1 bis 5, **dadurch gekennzeichnet, dass** die Membran (3) aus einem medizinischen Silikon und/oder vergleichbaren Softmaterialien in der Härte von 20 Shore A bis 50 Shore A besteht und eine Dicke von 0,5 mm bis 1,0 mm aufweist.

7. Phonationsventil nach mindestens einem der Ansprüche 1 bis 6, **dadurch gekennzeichnet, dass** zwischen dem Rand der Membran (3) und dem inneren Rand des Tragelementes (1) ein Spalt (6) vorgesehen ist.

8. Phonationsventil nach mindestens einem der Ansprüche 1 bis 7 **dadurch gekennzeichnet, dass** das Stützgerüst (2) aus einem medizinisch zugelassenem Photopolymer oder einem anderen harten Kunststoff oder einem Medical Grade Silikon mit der Härte 50 Shore A bis 20 Shore D besteht.

9. Phonationsventil nach mindestens einem der Ansprüche 1 bis 8, **dadurch gekennzeichnet, dass** das Tragelement (1) aus einem Silikon medizinischer Qualität mit einer Shore-Härte von 50 Shore A bis 20 Shore D besteht.

10. Phonationsventil nach mindestens einem der Ansprüche 1 bis 9, **dadurch gekennzeichnet, dass** es im 3D-Druck hergestellt ist.
